# EUROPEAN PATENT APPLICATION

(11) **EP 4 825 987 A2**
(43) Date of publication of application: **21.10.2026**
(21) Application number: 26202659.4
(22) Date of filing: 03.08.2023
(51) Int. Cl.: C12R 1/84

(54) **HIGH-STABILITY RECOMBINANT COLLAGEN, AND CONSTRUCTION METHOD THEREFOR AND USE THEREOF**

(30) Priority: 10.08.2022 CN 202210954027
(62) Document number(s) of the earlier application(s) in accordance with Art. 76 EPC: 23851688.4 / 4 567 044
(71) Applicant: Jiangsu Trautec Medical Technology Co., Ltd., Changzhou, Jiangsu 213251 (CN)
(72) Inventor: WANG, Liping, Changzhou, Jiangsu, 213251 (CN); LIU, Xiaohai, Changzhou, Jiangsu, 213251 (CN); QIAN, Chenming, Changzhou, Jiangsu, 213251 (CN); CHENG, Pengfei, Changzhou, Jiangsu, 213251 (CN); DOU, Rongkun, Changzhou, Jiangsu, 213251 (CN); FAN, Xiaoju, Changzhou, Jiangsu, 213251 (CN); QIAN, Song, Changzhou, Jiangsu, 213251 (CN)
(74) Representative: Lorenz Seidler Gossel Part. mbB

(57) **Abstract**

Disclosed in the present invention are a high-stability recombinant collagen, and a construction method therefor and the use thereof. On the basis of an original collagen sequence, one or more mutations selected from a group consisting of avoiding an unstable GXY triplet, removing potential MMP cleavage sites, eliminating unstable chemical factors, and increasing the content of RGD in the sequence are performed. The method can be extended to expression systems other than Pichia pastoris. The recombinant collagen obtained by means of the method has physicochemical properties and biological functions similar to those of the original sequence. When used as a biological material, the recombinant collagen has high inter-batch consistency, is convenient for quality control and facilitates product stability due to having higher stability.

## Description

### TECHNICAL FIELD

The present invention relates to the technical field of collagens, and in particular to a high-stability recombinant collagen, and a construction method therefor and use thereof.

### BACKGROUND

A collagen, as the most abundant type of protein in an animal body and an important extracellular matrix component, plays an important role in cell migration, cell metabolism, cell signaling pathway response, platelet aggregation, as well as maintenance, regulation and damage repair of normal physiological functions of cells, tissues and organs, etc. The collagen has good biocompatibility, biological activity and degradability, and has been widely used in many fields such as chemicals, medicines, foods and cosmetics.

The technology of extracting the collagen from an animal tissue has been relatively mature, and such collagen has a long history of application. However, raw materials such as acid and alkali used in a traditional production process of the collagen are not friendly to the environment. An extracted collagen peptide is uneven in nature, has a large difference between batches, and has a potential safety hazard of viral infection. For such reasons, a recombinant collagen produced by genetic engineering technology has attracted more and more attention. The research and application of the recombinant collagen have a history of more than 30 years. Existing literature and patents mainly focused on the expression of a human-source collagen single chain and a truncated collagen gene single chain in different hosts. In addition, a small amount of attention was paid to the collagen for obtaining a triple helix structure by co-expression of the collagen and a related post-expression modification enzyme .

The expression of the recombinant collagen involved in the prior art mostly uses human-source collagen sequences (CN201010527766.7, CN201510535383.7 and CN201911093124.8). Most of expression products are random coils and have less regular secondary structures, and fewer high-level structures such as tertiary and quaternary structures. In addition, as a water-soluble protein is easy to be processed and used, an amino acid sequence tends to be selected to be highly water-soluble. As a result, such a linear recombinant collagen is easily degraded by a protease within a host. During purification and processing, a highly water-soluble protein cannot be folded into a stable high-level structure due to a small content of hydrophobic regions. Unstable side chains, residues and peptide bonds are susceptible to deamidation, oxidation and hydrolysis, which may eventually lead to production of process-related impurities and degradation products, thus increasing immunogenicity.

It is an urgent need in this art to obtain a high-stability recombinant collagen.

### SUMMARY

Thew present invention is defined by the independent claims. Embodiments of the invention are recited in the dependent claims. To overcome the above problems, the present invention aims to obtain a recombinant collagen sequence with improved stability by mutation of an existing amino acid sequence or selection of an amino acid sequence, thus simplifying a purification process, and improving protein yield and storage stability.

Specifically, a first aspect of the present invention provides a construction method of a collagen variant with improved stability, including the following step: on basis of an original collagen sequence, performing one or more mutations selected from a group consisting of avoiding an unstable GXY triplet, removing a potential MMP cleavage site, eliminating an unstable chemical factor, and increasing content of RGD in the sequence.

In some embodiments, an original collagen includes an amino acid sequence as set forth in SEQ ID NO: 1 or includes an amino acid sequence with over 80%, over 85%, over 90%, over 95%, over 96%, over 97%, over 98% and over 99% identity with SEQ ID NO: 1.

In some embodiments, G is glycine in the GXY triplet. X and Y can be arbitrary amino acids. A collagen consists of a GXY motif.

In some embodiments, avoiding the unstable GXY triplet includes performing mutations selected from GPL→GPS, GIA→GPA, GDR→GER and GAA→ GPA in an initial collagen sequence.

In some embodiments, removing the potential MMP cleavage site includes removing a potential MMP2 cleavage site and/or a potential MMP9 cleavage site; preferably, performing the mutation of GLA→GPA and/or a mutation of GIK→GEK.

In some embodiments, the unstable chemical factor includes a site that is easily deamidated, hydrolyzed or oxidized; preferably, performing mutations selected from the following: 1) NG→QG; 2) F→P; 3) MPGPR→PPGPR; 4) a mutation from other R to K while retaining an original RGD sequence; 5) a mutation from other D to E while retaining the original RGD sequence.

A second aspect of the present invention provides a collagen variant with improved stability obtained on the basis of the method in the first aspect of the present invention.

In some embodiments, a sequence of the collagen variant includes an amino acid sequence selected with over 80%, over 85%, over 90%, over 95%, over 96%, over 97%, over 98% and over 99% identity with SEQ ID NO:2, SEQ ID NO:3, SEQ ID NO:4 or SEQ ID NO:6.

In some embodiments, the sequence of the collagen variant includes an amino acid sequence that is selected and as set forth in SEQ ID NO:2, SEQ ID NO:3, SEQ ID NO:4 or SEQ ID NO:6.

A third aspect of the present invention provides a high-stability recombinant collagen, including an amino acid sequence that is selected and as set forth in SEQ ID NO:2, SEQ ID NO:3, SEQ ID NO:4 or SEQ ID NO:6, or an amino acid sequence selected with over 80%, over 85%, over 90%, over 95%, over 96%, over 97%, over 98% and over 99% identity with SEQ ID NO:2, SEQ ID NO:3, SEQ ID NO:4 or SEQ ID NO:6.

A fourth aspect of the present invention provides an isolated polynucleotide. The polynucleotide encodes the collagen variant with the improved stability according to the second aspect of the present invention or the high-stability recombinant collagen according to the third aspect of the present invention.

In some embodiments, the polynucleotide includes a nucleic acid sequence selected with over 80%, over 85%, over 90%, over 95%, over 96%, over 97%, over 98% and over 99% identity with SEQ ID NO:7, SEQ ID NO:8, SEQ ID NO:9 or SEQ ID NO:10.

In some embodiments, the polynucleotide includes a nucleic acid sequence that is selected and as set forth in SEQ ID NO:7, SEQ ID NO:8, SEQ ID NO:9 or SEQ ID NO:10.

A fifth aspect of the present invention provides a vector, including the isolated polynucleotide according to the fourth aspect of the present invention.

In some embodiments, the vector is a eukaryotic vector.

In some embodiments, the vector is a prokaryotic vector.

The fifth aspect of the present invention provides a host cell, including the isolated polynucleotide according to the fourth aspect of the present invention or the vector according to the fifth aspect of the present invention.

In some embodiments, the host cell is a eukaryotic cell. Preferably, the host cell is a pichia pastoris. More preferably, the pichia pastoris is deposited in China General Microbiological Culture Collection Center with accession numbers of CGMCC No.24687, CGMCC No.24688, CGMCC No.24689, and CGMCC No.24690.

In some embodiments, the host cell is a prokaryotic cell. Preferably, the prokaryotic cell is a genetically engineered bacterium.

A sixth aspect of the present invention provides a composition, including the collagen variant with the improved stability according to the second aspect of the present invention or the high-stability recombinant collagen according to the third aspect of the present invention.

A seventh aspect of the present invention provides a product, including the collagen variant with the improved stability according to the second aspect of the present invention, the high-stability recombinant collagen according to the third aspect of the present invention, and the composition provided in the sixth aspect of the present invention.

An eighth aspect of the present invention provides use of the collagen variant with the improved stability according to the second aspect of the present invention, the high-stability recombinant collagen according to the third aspect of the present invention, the composition provided in the sixth aspect of the present invention, or the product according to the seventh aspect of the present invention in preparation of a medical device, a biomaterial, a tissue engineering product or a cosmetic.

Compared with the prior art, the present invention has the following beneficial effects.
(1) The present invention provides a construction method of a high-stability collagen variant, including the following steps: avoiding an unstable GXY triplet, removing a potential MMP cleavage site, eliminating an unstable chemical factor, and increasing content of RGD in a sequence. This method can be extended to an expression system other than a pichia pastoris.
(2) The recombinant protein involved in the present invention has improved stability. The main band accounts for more than 85% in a fermentation stage. The recombinant protein is not easy to degrade in a purification process, which greatly improves the yield of a full-length protein;
(3) The sample obtained after purification of the recombinant protein involved in the present invention performs better than an original protein in a stability experiment. An aqueous solution does not show obvious degradation band after being stored at 4°C for 1 month. A lyophilized sponge can be stored at room temperature for a long time, which reduces storage difficulty and prolongs storage time;
(4) The recombinant protein involved in the present invention has similar physicochemical properties and biological functions to an original sequence. When used as a biological material, the recombinant protein facilitates quality control and is conducive to product stability due to higher stability and high consistency between batches.

### BRIEF DESCRIPTION OF THE DRAWINGS

Other features, purposes and advantages of the present invention will become more apparent by reading the detailed description of the non-limiting embodiments with reference to the following drawings:
FIG. 1 shows mass spectrometry detection results of A recombinant collagen.
FIG. 2 shows SDS-PAGE of expression supernatant (induced 48 h) of a shake flask of a recombinant collagen.
FIG. 3 shows an SDS-PAGE diagram of a fermentation supernatant of 5L tank of each recombinant collagen.
FIG. 4 shows SDS-PAGE of a recombinant collagen sponge.
FIG. 5 shows M4 reversed-phase chromatography results of a recombinant collagen sponge.
FIG. 6 shows the stability of a recombinant collagen liquid at different temperatures.
FIG. 7 shows the stability of a recombinant collagen liquid at different pHs.
FIG. 8 shows the detection of cell adhesion activity.

### DETAILED DESCRIPTION OF THE EMBODIMENTS

In order to make the purpose, technical solutions and advantages of embodiments of the present invention clearer, the technical solutions of the embodiments of the present invention will be clearly and completely described below in conjunction with drawings of the embodiments of the present invention. Apparently, the described embodiments are some embodiments of the present invention, not all embodiments. Based on the described embodiments of the present invention, all other embodiments obtained by a person skilled in the art without creative labor fall within the scope of protection of the present invention.

Unless otherwise defined, technical or scientific terms used herein shall have the ordinary meaning understood by a person skilled in the art to which the invention belongs.

The present invention provides a method for preparing a high-stability collagen variant, identification of properties of the collagen variant obtained by the method and use thereof. The method includes the following steps:

### (1) Designing a collagen variant sequence:

A series of variants are designed based on a collagen sequence in CN201310033299.6 (a patented amino acid sequence is selected from a Type-3 collagen). M2 changes an unstable GXY triplet (Bachinger, H. P. and J. M. Davis, Sequence specific thermalstability of the collagen triple helix. Int J Biol Macromol, 1991.13(3)::p.152-6.). A potential MMP cleavage site is removed. NG of an easily deamidated site is mutated to QG. Photooxidation sensitivity F is mutated to P and has a 95% homology with an original sequence. M4, based on M2, retains an original RGD sequence. Other R is mutated to K and has a 91.7% homology with the original sequence. M5, based on M2, retains the original RGD. Other D is mutated to E and has a point mutation so that a total sequence includes 8 RGD motifs (MPGPR → PPGPR) and has a 92% homology with the original sequence. A stable sequences without the above unstable GXY triplets, the potential MMP cleavage site, and the unstable chemical factor are selected from the original sequence and spliced to form a monomer, and M6 is formed after repetition for 5 times.

### (2) Constructing a recombinant expression vector:

A DNA sequence including M2, M4, M5 and M6 is synthesized. An exogenous DNA is connected to an expression vector pPIC9K to construct the recombinant expression vector expressing M2, M4, M5 and M6 respectively.

### (3) Constructing a recombinant engineering strain, induced expressing, and screening the strain;

A recombinant expression vector is linearized in PmeI. A pichia pastoris competent cell is electrotransferred, and coated to an MD plate. After primary screening, the pichia pastoris competent cell is screened via YPD plates including different concentrations of G418. A selected bacterial colony is inoculated into a BMGY medium, and then induces expression via a BMMY medium. A plurality of strains are selected. One engineering strain with high expression is selected for subsequent experiments.

### (4) Identifying a protein expression;

An expressed protein is preliminarily identified via SDS-PAGE electrophoresis. It is indicated that: M2, M4, M5 and M6 can be efficiently secreted and expressed outside a cell. A target band accounts for more than 90%, with few degradation fragments, and the size consistent with apparent migration characteristics of a collagen. The results of liquid chromatography analysis are consistent with SDS-PAGE. The target band has more than 90% content.

### (5) Fermenting under a high density , culturing, and purifying a protein;

The high-density fermentation experiment is carried out using a fermentation tank. A main band verified by SDS-PAGE accounts for more than 85%. The target band obtained after one-step cation exchange and purification accounts for up to 95%.

### (6) Testing the cell adhesion activity of a recombinant collagen through an in vitro cell experiment;

An in vitro cultured NIH/3T3 cell is used for cell adhesion of M2, M4, M5 and M6 to the original sequence. Experiment results indicate that the adhesion activity of M2, M4, M5 and M6 are not apparently different from that of the original sequence and is basically the same as that of a commercial human collagen.

### (7) Testing the stability of the recombinant collagen;

With an original sequence protein as control, the stability of a lyophilized sponge of recombinant proteins M2, M4, M5 and M6 is detected at different temperatures (-20°C, 4°C, 25°C, 40°C, 60°C) and pHs (4, 7, 9). SDS-PAGE and liquid phase are used to monitor a content change of the target band. Stability of M2, M4, M5 and M6 is significantly higher than that of the original sequence.

### (8) Testing the safety of the recombinant collagen;

CCIC Huatongwei International Inspection (Suzhou) Co., Ltd. was entrusted to test the potential cytotoxic effect of a recombinant collagen M4 using mammalian L-929 cells cultured in vitro in accordance with method requirements of GB/T16886.5-2017. According to the method requirements of GB/T16886.10-2017, a potential intradermal reaction test of the recombinant collagen M4 was observed using a New Zealand rabbit. According to the method requirements of GB/T16886.10-2017, the potential skin sensitization effect of the recombinant collagen M4 was observed using the maximum dose test method of a guinea pig. Results showed that there was no cytotoxicity, intradermal reactions or skin sensitization effect.

### (9) Preparing and detecting a bladder repair agent of the recombinant collagen

The high-stability recombinant collagen, a production method therefor and performance verification of the present invention are elaborated in conjunction with specific embodiments in the following.

### Embodiment 1: Design of an amino acid sequence of a collagen variant

An original sequence was a sequence in CN201310033299.6, which is connected in series with No. 908-1136AA in a Type-3 collagen (https://www.uniprot.org/uniprot/P02461) sequence as a monomer. Terminal C is fused with 6His tag, with a sequence of SEQ ID NO:1:

AGNTGAPGSPGVSGPKGDAGQPGEKGSPGAQGPPGAPGPLGIAGITGARGLAGPPGMP
GPRGSPGPQGVKGESGKPGANGLSGERGPPGPQGLPGLAGTAGEPGRDGNPGSDGLPG
RDGSPGGKGDRGENGSPGAPGAPGHPGPPGPVGPAGKSGDRGESGPAGPAGAPGPAGS
RGAPGPQGPRGDKGETGERGAAGIKGHRGFPGNPGAPGSPGPAGQQGAIGSPGPAEFTA
GNTGAPGSPGVSGPKGDAGQPGEKGSPGAQGPPGAPGPLGIAGITGARGLAGPPGMPG

PRGSPGPQGVKGESGKPGANGLSGERGPPGPQGLPGLAGTAGEPGRDGNPGSDGLPGR
DGSPGGKGDRGENGSPGAPGAPGHPGPPGPVGPAGKSGDRGESGPAGPAGAPGPAGSR
GAPGPQGPRGDKGETGERGAAGIKGHRGFPGNPGAPGSPGPAGQQGAIGSPGPADHHH
HHHTGLARF

M2 changed a triplet with a stability score of 0.0 to a triplet with a score of 2.0 or 1.0. Specific change sequence was as follows: GPLGIA (38-43, 270-275)→GPSGPA, GDR (125-127, 155-157, 357-359, 387-389) → GER, GAA (194-196, 426-428) → GPA. D462, connection sequence EFT (230-232) and 6His post-sequence TGLARF (469-474) were deleted. GLA (potential MMP2 and MMP9 recognition sites, 49-51, 94-96, 277-279, 322-324) → GPA was mutated. GIK (potential MMP2 recognition site, 196-198, 424-426) → GEK was mutated. A unstable chemical factor was removed, such as NG (sites prone to deamidation, 78-79, 129-130, 306-307, 357-358)→QG, F (light-induced oxidation sensitivity, 203, 431) →P. A sequence thereof was shown as SEQ ID NO:2:

AGNTGAPGSPGVSGPKGDAGQPGEKGSPGAQGPPGAPGPSGPAGITGARGPAGPPGMP
GPRGSPGPQGVKGESGKPGAQGLSGERGPPGPQGLPGPAGTAGEPGRDGNPGSDGLPG
RDGSPGGKGERGEQGSPGAPGAPGHPGPPGPVGPAGKSGERGESGPAGPAGAPGPAGS
RGAPGPQGPRGDKGETGERGPAGEKGHRGPPGNPGAPGSPGPAGQQGAIGSPGPAGNT
GAPGSPGVSGPKGDAGQPGEKGSPGAQGPPGAPGPSGPAGITGARGPAGPPGMPGPRG
SPGPQGVKGESGKPGAQGLSGERGPPGPQGLPGPAGTAGEPGRDGNPGSDGLPGRDGS
PGGKGERGEQGSPGAPGAPGHPGPPGPVGPAGKSGERGESGPAGPAGAPGPAGSRGAP
GPQGPRGDKGETGERGPAGEKGHRGPPGNPGAPGSPGPAGQQGAIGSPGPAHHHHHH

M4, based on M2, retained an original RGD sequence, and mutated other R to K (a variety of endogenous proteases in a pichia pastoris identified a basic amino acid, and were cleaved at Terminal C. The most common basic amino acid at a MMP cleavage site is arginine). A sequence thereof was shown as SEQ ID NO:3:

AGNTGAPGSPGVSGPKGDAGQPGEKGSPGAQGPPGAPGPSGPAGITGAKGPAGPPGMP
GPKGSPGPQGVKGESGKPGAQGLSGEKGPPGPQGLPGPAGTAGEPGKDGNPGSDGLPG
KDGSPGGKGEKGENGSPGAPGAPGHPGPPGPVGPAGKSGEKGESGPAGPAGAPGPAGS
KGAPGPQGPRGDKGETGEKGPAGEKGHRGPPGNPGAPGSPGPAGQQGAIGSPGPAGNT

GAPGSPGVSGPKGDAGQPGEKGSPGAQGPPGAPGPSGPAGITGAKGPAGPPGMPGPKG
SPGPQGVKGESGKPGAQGLSGEKGPPGPQGLPGPAGTAGEPGKDGNPGSDGLPGKDGS
PGGKGEKGENGSPGAPGAPGHPGPPGPVGPAGKSGEKGESGPAGPAGAPGPAGSKGAP
GPQGPRGDKGETGEKGPAGEKGHRGPPGNPGAPGSPGPAGQQGAIGSPGPA

M5, based on M2, retained the original RGD sequence, and mutated other D to E (glutamine was prone to hydrolysis). With a point mutation, a total sequence includes 8 RGD motifs, MPGPR → PPGPR (M was located on the -5 position of a Kex2 identification site. -4 position was P that hindered MMP cleavage. M was prone to oxidation, thus leading to mutation). A sequence thereof was shown as SEQ ID NO:4:

AGNTGAPGSPGVSGPKGEAGQPGEKGSPGAQGPPGAPGPSGPAGITGARGDAGPPGpP
GPRGSPGPQGVKGESGKPGAQGLSGERGDPGPQGLPGPAGTAGEPGREGNPGSEGLPG
REGSPGGKGERGDQGSPGAPGAPGHPGPPGPVGPAGKSGERGESGPAGPAGAPGPAGS
RGAPGPQGPRGDKGETGERGPAGEKGHRGPPGNPGAPGSPGPAGQQGAIGSPGPAGNT
GAPGSPGVSGPKGEAGQPGEKGSPGAQGPPGAPGPSGPAGITGARGDAGPPGpPGPRGS
PGPQGVKGESGKPGAQGLSGERGDPGPQGLPGPAGTAGEPGREGNPGSEGLPGREGSP
GGKGERGDQGSPGAPGAPGHPGPPGPVGPAGKSGERGESGPAGPAGAPGPAGSRGAPG
PQGPRGDKGETGERGPAGEKGHRGPPGNPGAPGSPGPAGQQGAIGSPGPAHHHHHH

Sequences (2-37aa, 62-85aa, 134-154aa, 176-190aa) without the MMP cleavage site and an unstable triplet in the original sequence were selected and spliced to form a monomer. A sequence thereof was shown as SEQ ID NO:5:GNTGAPGSPGVSGPKGDAGQPGEKGSPGAQGPPGAPGSPGPQGVKGESGKPGA NGLSGENGSPGAPGAPGHPGPPGPVGPAGKSGAPGPQGPRGDKGET

M6 was formed after SEQ ID NO.5 was repeated for five times. A sequence thereof was shown as SEQ ID NO: 6:

GPPGNTGAPGSPGVSGPKGDAGQPGEKGSPGAQGPPGAPGSPGPQGVKGESGKPGAN
GLSGENGSPGAPGAPGHPGPPGPVGPAGKSGAPGPQGPRGDKGETGNTGAPGSPGVSG
PKGDAGQPGEKGSPGAQGPPGAPGSPGPQGVKGESGKPGANGLSGENGSPGAPGAPG
HPGPPGPVGPAGKSGAPGPQGPRGDKGETGNTGAPGSPGVSGPKGDAGQPGEKGSPGA

QGPPGAPGSPGPQGVKGESGKPGANGLSGENGSPGAPGAPGHPGPPGPVGPAGKSGAP
GPQGPRGDKGETGNTGAPGSPGVSGPKGDAGQPGEKGSPGAQGPPGAPGSPGPQGVK
GESGKPGANGLSGENGSPGAPGAPGHPGPPGPVGPAGKSGAPGPQGPRGDKGETGNTG
APGSPGVSGPKGDAGQPGEKGSPGAQGPPGAPGSPGPQGVKGESGKPGANGLSGENGS
PGAPGAPGHPGPPGPVGPAGKSGAPGPQGPRGDKGETGG

Polynucleotide sequences encoded with M2, M4, M5, and M6 were as set forth in SEQ ID NO:7-10, respectively.

### Embodiment 2: Synthesis of a DNA sequence and construction of a recombinant expression vector

GenScript Biotech Corporation was entrusted to synthesize a DNA fragment expressing M2, M4, M5 and M6, and clone a synthesized gene fragment into a pPIC9K empty vector (purchased from Thermo Fisher Scientific), so that a target fragment was accurately inserted into a secreted type vector reading frame including a secreted signal α-factor to obtain recombinant plasmids expressing M2, M4, M5 and M6.

### Embodiment 3: Construction of a recombinant engineering strain and strain screening

10 µg of the above recombinant expression plasmid was digested with PmeI (purchased from Dalian TaKaRa Company. Specific operation was carried out according to kit instructions) MMP cleavage at 37°C overnight for linearization, and then a PCR product purification kit (purchased from Sangon Biotech (Shanghai) Co., Ltd.) was used to recover a linearized plasmid, and control a volume at about 10 µL.

The linearized plasmid was electrotransferred into a pichia pastoris SMD1168 (purchased from Thermo Fisher Scientific) competent cell of a host cell. An electrotransferred bacterial solution was coated on an MD plate. One plate was coated each 100 µL-200 µL, standed at room temperature for 10 min, and was inverted and incubated at 30°C for 2-5 days until a single bacterial colony (a positive transformant) appeared.

2 mL of sterile double-distilled water was added to the surface of the MD plate. Then, the His+ transformant on the surface of the plate was gently scraped off with a sterile triangular applicator, and transferred to a 50-mL centrifuge tube. A bacterial suspension was diluted with the sterile double distilled water. 10⁵ cells were coated on a YPD plate including 0.5 mg/mLG418. The YPD plate was inverted and incubated at 30°C for 3-4 days until the single bacterial colony appeared. The bacterial colony was selected from the YPD plate, put into a sterile 96-well plate (200 µL YPD/well), mixed well, and incubated at 30°C for 48 h. A bacterial solution was mixed evenly in wells. 10 µL of the bacterial solution was inoculated to a new sterile 96-well plate, and cultured at 30°C for 24 h. This operation was repeated again. After 24 h, 1 µL of the bacterial solution was taken from a third 96-well plate, applied on the YPD plate including 1.0 mg/mL and 4 mg/mL G418, respectively, and continued to be cultured at 30°C for 96 h-120 h. If the pichia pastoris transformant could be grown on a plate including a high concentration of G418, it indicated that the transformant included a plurality of copies of a gene of interest, that is, a plurality of recombinant fragments entered a yeast, and were integrated into chromosomes of the yeast through homologous recombination. After screening in this step, a recombinant yeast engineering strain with a high copy and highly efficient expression could be obtained.

Samples of 4 engineered bacteria constructed were sent to China General Microbiological Culture Collection Center, with accession numbers of No.24687, No.24688, No.24689, No.24690. Address: No.3, Yard 1, Beichen West Road, Chaoyang District, Beijing; deposited date: April 18, 2022. Classification naming: pichia pastoris *Komagataella phaffii*.

### Embodiment 4: Induced expression and identification of a recombinant collagen

Engineering strains of a pichia pastoris expressing M2, M4, M5 and M6 were taken, respectively. At the same time, engineering strains in patent CN201310033299.6 were taken, placed in a 100-mL triangular flask filled with 10mLBMGY medium, and cultured at 28-30°C and 220 rpm until OD600 was 2-6 (16-18h). The engineering strains of the pichia pastoris were centrifuged at 1500-3000 g at room temperature for 5 min. A thallus was collected, and resuspended with a BMMY medium so that the OD₆₀₀ was about 2. The thallus was placed on a shaker at 28-30°C and 220rpm to continue to grow for 3 days. 100% methanol was added to the medium every 24 h until a final concentration was 1.0%. The methanol was added and induced for more than 16 h. A bacterial liquid sample was collected with a sampling amount of 1 mL, placed in a 1.5 mL EP tube, and centrifuged at 12000 g at 4°C for 5 min. An expression supernatant was collected. The sample to be tested was stored at -80°C for later use.

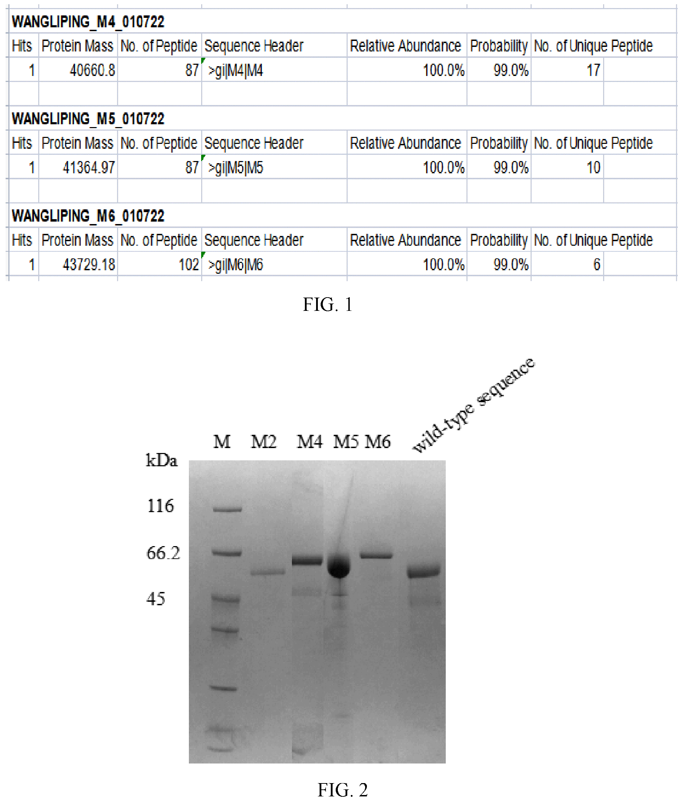

The collected expression supernatant was added to a 5 × sample loading buffer (250 mM Tris-HCl, pH 6.8, 10% SDS, 0.5% bromophenol blue, 50% glycerol, 5% β-mercaptoethanol), and heated in a 100°C metal bath for 10 min for SDS-PAGE detection. Results were as set forth in FIG. 2. All collagen variants could be efficiently secreted into a medium supernatant. A target band accounted for more than 80%.

An expected band of M4, M5 and M6 on SDS-PAGE was cut down and performed with enzymolysis with trypsin. A peptide segment of the recombinant collagen performed with enzymolysis with the trypsin was detected with Nano-HPLC-MS/MS mass spectrometry (completed by Suzhou Putai Biotechnology Co., Ltd.). The detected peptide segment was compared with a theoretical sequence. As as set forth in FIG. 1, The peptide segments detected after enzymolysis of M4, M5 and M6 all belonged to the theoretical sequences of the corresponding collagen variant, indicating that each collagen variant was successfully expressed.

### Embodiment 5: High-density fermentation and purification tests

(1) A high-density fermentation experiment was performed on a genetically engineered bacterium. A large-scale expression and production were performed with recombinant M2, M4, M5 and M6 collagens to obtain a fermentation broth including the recombinant collagen.

A seed medium (yeast powder of 10 g/L, peptone of 20 g/L and glycerol of 10 g/L); a fermentation medium (NH₄H₂PO₄ of 190.4 g/L, KH₂PO₄ of 10.06 g/L, CaSO₄·2H₂O of 1.18 g/L, K₂SO₄ of 18.2 g/L, MgSO₄·7H₂O of 14.9 g/L and glycerol of 40 g/L); a supplemental medium (50% W/V glycerol was added with 12 mL PTM1 trace elements per liter); an induction medium (100% methanol was added with 12 mLPTM1 trace elements per liter); PTM1: Sterilization was performed by filtration with a 0.22 µm filter membrane, and storage was performed at 4°C. After the fermentation medium was sterilized at high temperature, and the temperature is reduced to room temperature, PTM1 was added. PH was adjusted to 5.0 with ammonia water.

The batch culture conditions and induced expression conditions of an engineering strain were as follows: A fed-batch culture method was used with a culture temperature of 30°C. The engineering bacterium was inoculated into a 1 L shake flask including 200 mL of a seed medium YPG and cultured at 220 rpm and 30°C for 18-20h until 0D600=2-10. A 5 L fermentation tank (Shanghai Baoxing Biological Equipment Engineering Co., Ltd.) was filled with the fermentation medium of 2L and 2% glycerol for isolated sterilization. A rotation speed was adjusted to 300rpm before inoculation, with a ventilation volume of 4 L/min and a temperature of 30°C. pH was adjusted with an alkaline solution prepared with concentrated ammonia water, and set to 4.5. Then, 0.9mL of PTM1 was first inoculated. 200 mL of a prepared seed liquid was inoculated into the tank (flame circle inoculation). A dissolved oxygen electrode was then clicked to calibrate 100, and then started fermentation. When a grown dissolved oxygen fell to 30% for the first time, a dissolved oxygen cascade speed function was used to maintain 30%. Glycerin was waited for to be exhausted, the dissolved oxygen rebounded, and was greater than 70% (an OD₆₀₀ value was about 20). The dissolved oxygen cascade speed was cancelled. A stirring rotation speed was increased to 650rpm. 30% linkage feeding material (80 mL) was used for glycerin. After glycerol was stopped from feeding, the dissolved oxygen rebounded to more than 70%. pH was set to 4, and the temperature was set to 29°C. Induced culture was performed with a carbon source mixed with methanol and glycerol (methanol: 50%, glycerol = 7:3). 5 mL of the carbon source was manually added . After the dissolved oxygen rebounded to more than 70%, a feeding speed was set to 8 mL/h, increased to 10 mL/h after 1 h, and increased again to 20 mL/h after 1 h. When a dissolved oxygen value was lower than 30%, feeding was stopped. The dissolved oxygen was waited for to rebound. The linkage feeding was performed after the dissolved oxygen rose to 30%. After induction for 40-60 h, a protein concentration measured by UV was not obviously increased or decreased, the tank can be put down. UV protein quantification formula: (C(mg/mL)=0.144*(A215-A225), A215<1.5.

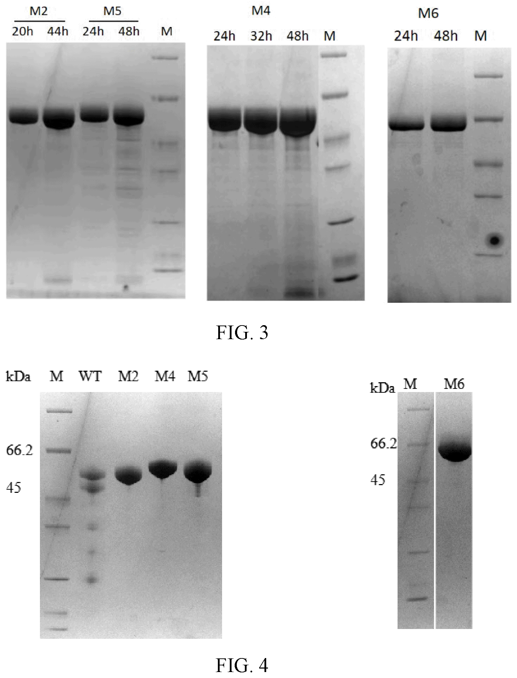

A fermentation supernatant was collected for SDS-PAGE electrophoresis detection, as as set forth in FIG. 3. Under the condition of high-density fermentation, each variant collagen had almost only a target band within 24 h of induction, M4 and M5 were slightly degraded at 48 h. A main band accounted for more than 85% through the optical density analysis.

### (2) Purification

Buffer A: 20 mM MHH₂PO4, pH 4.0;
Buffer B: 20mMKH₂PO4, 1MNaCl, pH4.0.

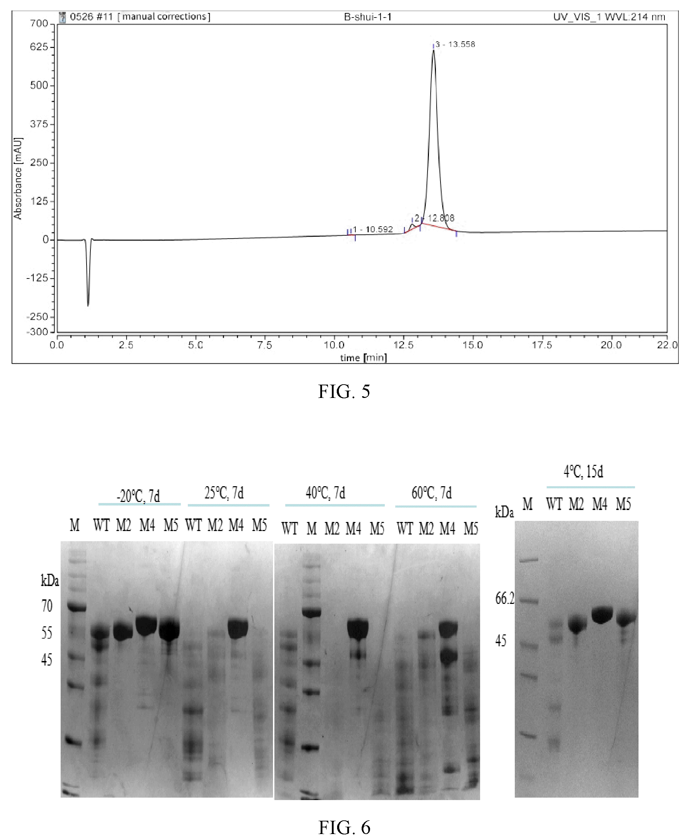

A fermentation broth was collected. A thallus and a fermentation supernatant were centrifuged at 2000g and 4°C for 30 min. After a cation exchange medium (a chromatography packing was UniGel-80sp produced by Suzhou NanoMicro Technology Co Ltd, was loaded on an XK50/30 chromatography column produced by Lisure Science (Suzhou) Co., Ltd. A GEAKTAPure protein separation chromatography purification system was used) was equilibrated with the buffer A until an absorbance value and a conductivity value of A215 remained unchanged, a flow rate of 100 us/cm was set to load a sample, with a loading volume of 0.5 L/time. An absorbance value of UV A215 was detected. When the absorbance value of UV A215 rose, sampling started. At the end of sample loading, sampling was closed. A cation chromatography medium was equilibrated with the buffer A. When the absorbance value of A215 decreased, sampling was opened until UV and conductance were minimized and no longer changed. Sampling was stopped. The buffer B was used to elute. After collected, detected and determined with the components, respectively, an eluent was dialysed (a dialysate was ultrapure water), concentrated, frozen and dried. A lyophilized collagen sponge was collected. The purified lyophilized sponge was dissolved in the ultrapure water, and performed with SDS-PAGE electrophoresis, as as set forth in FIG. 4. Image Lab software (Bio-Rad Gel DocXR + imager) was used for calculation: The purity of M2, M4, M5 and M6 was up to 97.1%, 96.7%, 90.6% and 97.6% respectively. The purity of M4 was determined to be 99.78% by reversed chromatography. Results were as set forth in FIG. 5.

### Embodiment 6: test for the stability of a recombinant collagen

UP water was added to lyophilized sponges of recombinant proteins M2, M4, M5 and M6 to prepare a 1 mg/mL solution, which was filtered by a 0.22 µm filter membrane, packed in a sterile centrifuge tube, placed at 60°C, 40°C, 25°C, 4°C and -20°C, respectively, and sampled on Day 3, Day 7 and Day 15 for SDS-PAGE detection. Results were as set forth in FIG. 6. There is no significant difference between each recombinant collagen placed at -20°C and 4°C and the initial one. The stability of M4 under liquid conditions at 60°C, 40°C and 25°C was significantly higher than that of an original sequence and other variants.

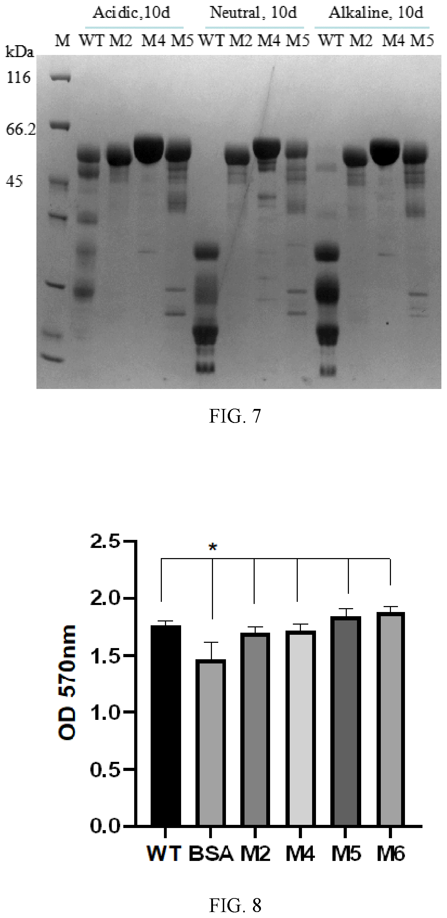

UP water was added to the lyophilized sponges of the recombinant proteins M2, M4, M5 and M6 to prepare the 1 mg/mL solution. pH was adjusted to acidic (pH 4-5), neutral (pH 7-7.5) and alkaline (pH 9-10). The solution was filtered by the 0.22 µm filter membrane, packed in the sterile centrifuge tube, placed at 4°C, and sampled on Day 1, Day 5, Day 10, and Day 30 for SDS-PAGE detection. Only the test results of Day 10 were displayed. On Day 5, the recombinant collagen of the original sequence under neutral and alkaline conditions had no main band (the results were not shown). FIG. 7 showed that the results of the original sequence on Day 10 were consistent with those on Day 5. All of the original sequences were degraded. Main bands of other variants still accounted for more than 60%. The stability of each variant was significantly higher than that of the original sequence protein under neutral conditions.

### Embodiment 7: test for adhesion activity of a recombinant collagen cell

An NIH/3T3 cell (purchased from the Cell Bank of the Chinese Academy of Sciences, Item No. GNM6, and culture and passage methods were implemented according to cell instructions) was normally cultured. Lyophilized sponges of recombinant M2, M4, M5 and M6 collagens were taken. A human collagen (Sigma, Item No. C7774) and a bovine serum albumin (BSA, purchased from Sangon Biotech (Shanghai) Co., Ltd.) were compared to dissolve (ultrapure water or 1MHCl solution). The protein concentration was measured with UV protein quantification empirical formula: C(mg/mL)=0.144*(A215-A225). A protein was then diluted to 0.5 mg/mL with PBS (pH 7.4). 100 µL of various protein solutions and blank PBS solution were added to a 96-well cell culture plate for control, and standed for 60 min at room temperature. 10⁵ 3T3 cells with a good culture status were added to each well, and incubated at 37°C and 5% CO₂ for 60 min. Cells in the wells were cleaned with PBS for 4 times. An absorbance value of 570 nm was detected with a LDH detection kit (Roche, 04744926001) (refer to instructions for a specific operation).

Absorbance characterized the cell adhesion activity of a collagen sample: the higher the absorbance, the more cells with protein adherence, the higher the adhesion activity, and the more collagen could help cells adhere to a wall or adhere to an extracellular matrix of the cell in a short period of time, which is more conducive to building a suitable extracellular environment. As as set forth in FIG. 8, the adhesion activity of M2, M4, M5 and M6 was similar to that of the original sequence. There is no significant difference. All adhesion activity was significantly higher than that of a control group.

### Embodiment 8: test for the safety of a recombinant collagen

CCIC Huatongwei International Inspection (Suzhou) Co., Ltd. was entrusted to test the potential cytotoxic effect of a recombinant collagen M4 using mammalian L-929 cells cultured in vitro in accordance with method requirements of GB/T16886.5-2017. A test sample and a control sample were placed in a MEM medium including a 10% fetal bovine serum, and extracted in a 37°C incubator for 24 h. After the end of extraction, a cell medium is removed from ta 96-well plate (10⁴ cells/well) cultured for 24 h, replaced with the corresponding extraction liquid, and cultured in a cell culture incubator (37°C, 5% CO2, humidity >90% ) for 24 h. After the end of culture, the morphology and lysis of a cell was observed under a microscope. The cytotoxicity value of a test sample was measured using a MTT method. Results showed that cells in a blank control group and a negative control group (high-density polyethylene) were morphologically intact and normal throughout a test, and no cytotoxic reaction was shown. The severe cytotoxic reaction was as set forth in a positive control group (ZDEC). After incubation for 24 h, the cell morphology of the extraction liquid with a 100% concentration of the test sample was basically intact, and a cell viability value was 83.6%. The recombinant collagen M4 had no potential cytotoxic effect under the cytotoxicity test conditions of a MTT method.

CCIC Huatongwei International Inspection (Suzhou) Co., Ltd. was entrusted to observe a potential intradermal reaction test of the recombinant collagen M4 using a New Zealand rabbit according to method requirements of GB/T16886.10-2017. The sample was extracted using a 0.9% sodium chloride injection liquid and a sesame oil. A sufficient area of clothing hair on both sides of the animal's back spine was thoroughly removed 18 h before the test to prepare and inject the extraction liquid. On the day of the test, the liquid was injected intradermally at 10 sites on one side of the spine of each rabbit. 0.2 mL of a polar extraction liquid was injected at five points, and 0.2 mL of a non-polar extraction liquid was injected at five points. Polar and non-polar control solutions were injected on the other side of the spine with the same procedure as the extraction liquid of the test sample. According to the "Intradermal Reaction Scoring System", the conditions of each injection site were observed and recorded at (24±2) h, (48±2) h and (72±2) h. Results showed that animals did not show abnormal symptoms or died during the test. The skin reaction on one side of the animal in the test group did not exceed that of a negative control group. The skin of the animals in the test sample and the control group was intact. There are no reactions such as erythema and edema. The recombinant collagen M4 had no potential intradermal reaction in the New Zealand rabbit.

CCIC Huatongwei International Inspection (Suzhou) Co., Ltd. was entrusted to observe the potential skin sensitization effect of the recombinant collagen M4 using the maximum dose test method of a guinea pig according to method requirements of GB/T16886.10-2017. A sample was extracted using the 0.9% sodium chloride injection liquid and the sesame oil. A prepared extraction liquid was mixed with Freund's complete adjuvant to form a stable emulsifier. The emulsifier was injected intradermally into the depilated medial part of the dehaired scapula of each animal for intradermal induction and local induction. A provocative test was performed at the untested site of the animal in the induction stage 14 days after local induction. At 24 hours and 48 hours after the provocative test, skin reactions of the animals in a test group and a control group were observed at a provocative site. the erythema and edema reactions of skin at each provocative site were scored according to Magnusson and Kligman grading standards. Results showed that the animals in a negative control group (the 0.9% sodium chloride injection and the sesame oil) had intact skin and no skin erythema or edema reactions during the test. The animals in the positive control group (DNCB) showed significant skin erythema and edema reactions. The skin of the animals in a recombinant collagen M4 extraction liquid group was intact, and there was no skin erythema or edema reaction. The recombinant collagen M4 had no skin sensitization effect in the sensitization test.

### Embodiment 9: Preparation and detection of a bladder repair agent of a recombinant collagen

Formulas of the bladder repair agent of the recombinant collagen:
Specifications: 50 mL:0.5 g, concentration of 1%. Formulas: Recombinant collagen: 10 (g), sodium chloride: 9 (g), disodium hydrogen phosphate: 1 (g), water for injection: 1000 mL (20 bottles).

Preparation process: A prescription volume of water for injection was measured and placed in a liquid preparation tank. Prescription amounts of sodium chloride and disodium hydrogen phosphate were weighed, added to water, stirred for 15 min to completely dissolve. Then, a prescription amount of collagen was added, and stirred for 15 min to completely dissolve. A solution was passed through a 0.45 µm filter membrane, A filtrate was passed through a 0.22 µm filter membrane. The filtrate was packed (50 mL/bottle), melted and sealed.

Efficacy test: A model of interstitial cystitis of a mouse was constructed to evaluate the therapeutic effect of the repair agent of the recombinant collagen on cystitis. The study showed that the repair agent of the recombinant collagen could significantly improve bladder bleeding. After treated with the repair agent, the mucosal epithelium of a bladder can remained intact, without obvious edema and shedding damage. In addition, the submucosal collagen fibers of the bladder were tightly arranged, and a collagen content was significantly improved.

The above shows and describes the basic principles, main features and advantages of the present invention. For a person skilled in the art, it is obvious that the present invention is not limited to the details of the above-mentioned exemplary embodiments, and that the present invention can be realized in other specific forms without deviating from the spirit or basic features of the present invention. Therefore, the embodiments shall be regarded as exemplary and non-limiting in any way. The scope of the invention is limited by the appended claims rather than the above description. Therefore, all changes falling within the meaning and scope of the equivalent elements of the claims are intended to be included in the present invention. Any reference sign in the claims shall not be deemed to be a limitation of the claims concerned.

In addition, it shall be understood that not each embodiment contains only one independent technical solution although the specification is described according to the embodiments. The description method of the specification is only for clarity. A person skilled in the art shall take the specification as a whole. the technical solutions in each embodiment may also be appropriately combined to form other embodiments that can be understood by a person skilled in the art.
Aspects of the present invention can be formulated as follows:
1. A high-stability recombinant collagen, characterized in that the high-stability recombinant collagen is an amino acid sequence as set forth in SEQ ID NO:2, SEQ IDNO:3, SEQ ID NO:4 or SEQ ID NO:6.
2. The high-stability recombinant collagen according to aspect 1, characterized in that a construction method of the high-stability recombinant collagen comprises the following step: on basis of an original collagen sequence of SEQ ID NO:1, performing one or more mutations selected from a group consisting of avoiding an unstable GXY triplet, removing a potential MMP cleavage site, eliminating an unstable chemical factor, and increasing content of RGD in the sequence.
3. The high-stability recombinant collagen according to aspect 2, characterized in that avoiding the unstable GXY triplet comprises performing a mutation selected from GPL→GPS, GIA→GPA, GDR→GER and GAA→GPA in an initial collagen sequence; removing the potential MMP cleavage site comprises removing a potential MMP2 cleavage site and/or a potential MMP9 cleavage site; and the unstable chemical factor comprises a site that is easily deamidated, hydrolyzed or oxidized.
4. The high-stability recombinant collagen according to aspect 3, characterized in that removing the potential MMP cleavage site comprises performing a mutation of GLA→GPA and/or a mutation of GIK→GEK; the unstable chemical factor comprises performing a mutation selected from the following: 1) NG→QG; 2) F→P; 3) MPGPR→PPGPR; 4) a mutation from other R to K while retaining an original RGD sequence; 5) a mutation from other D to E while retaining the original RGD sequence.
5. An isolated polynucleotide encoding the high-stability recombinant collagen according to aspect 1.
6. The isolated polynucleotide according to aspect 5, characterized in that the polynucleotide is a nucleic acid sequence as set forth in SEQ ID NO:7, SEQ ID NO:8, SEQ ID NO:9 or SEQ ID NO:10.
7. A vector comprising the isolated polynucleotide according to aspect 5 or 6.
8. The vector according to aspect 7, characterized in that the vector is a eukaryotic vector or a prokaryotic vector.
9. A host cell comprising the isolated polynucleotide according to aspect 5 or 6 or the vector according to aspect 7 or 8.
10. The host cell according to aspect 9, characterized in that the host cell is a eukaryotic cell or a prokaryotic cell.
11. The host cell according to aspect 10, characterized in that the host cell is a *pichia pastoris*.
12. The host cell according to aspect 11, characterized in that the *pichia pastoris* is deposited in China General Microbiological Culture Collection Center with accession numbers of CGMCC No.24687, CGMCC No.24688, CGMCC No.24689, and CGMCC No.24690.
13. A composition comprising the high-stability recombinant collagen according to aspect 1.
14. A product comprising the high-stability recombinant collagen according to aspect 1 or the composition according to aspect 13.
15. Use of the high-stability recombinant collagen according to aspect 1, the composition according to aspect 13, or the product according to aspect 14 in preparation of a medical device, a biomaterial, a tissue engineered product or a cosmetic.

## Claims

1. A high-stability recombinant collagen, **characterized in that** the high-stability recombinant collagen is or contains an amino acid sequence as set forth in SEQ ID NO:6.

2. The high-stability recombinant collagen according to claim 1, **characterized in that** a construction method of the high-stability recombinant collagen comprises the following step: on basis of an original collagen sequence of SEQ ID NO:1, performing one or more mutations selected from a group consisting of avoiding an unstable GXY triplet, removing a potential MMP cleavage site, eliminating an unstable chemical factor, and increasing content of RGD in the sequence.

3. The high-stability recombinant collagen according to claim 2, **characterized in that** avoiding the unstable GXY triplet comprises performing a mutation selected from GPL→GPS, GIA→GPA, GDR→GER and GAA→GPA in an initial collagen sequence; removing the potential MMP cleavage site comprises removing a potential MMP2 cleavage site and/or a potential MMP9 cleavage site; and the unstable chemical factor comprises a site that is easily deamidated, hydrolyzed or oxidized.

4. The high-stability recombinant collagen according to claim 3, **characterized in that** removing the potential MMP cleavage site comprises performing a mutation of GLA→GPA and/or a mutation of GIK→GEK; the unstable chemical factor comprises performing a mutation selected from the following: 1) NG→QG; 2) F→P; 3) MPGPR→PPGPR; 4) a mutation from other R to K while retaining an original RGD sequence; 5) a mutation from other D to E while retaining the original RGD sequence.

5. An isolated polynucleotide encoding the high-stability recombinant collagen according to claim 1.

6. The isolated polynucleotide according to claim 5, **characterized in that** the polynucleotide is a nucleic acid sequence as set forth in SEQ ID NO:10.

7. A vector comprising the isolated polynucleotide according to claim 5 or 6.

8. The vector according to claim 7, **characterized in that** the vector is a eukaryotic vector or a prokaryotic vector.

9. A host cell comprising the isolated polynucleotide according to claim 5 or 6 or the vector according to claim 7 or 8.

10. The host cell according to claim 9, **characterized in that** the host cell is a eukaryotic cell or a prokaryotic cell.

11. The host cell according to claim 10, **characterized in that** the host cell is a *pichia pastoris*.

12. The host cell according to claim 11, **characterized in that** the *pichia pastoris* is deposited in China General Microbiological Culture Collection Center with accession numbers of CGMCC No.24690.

13. A composition comprising the high-stability recombinant collagen according to claim 1.

14. A product comprising the high-stability recombinant collagen according to claim 1 or the composition according to claim 13.

15. Use of the high-stability recombinant collagen according to claim 1, the composition according to claim 13, or the product according to claim 14 in preparation of a medical device, a biomaterial, a tissue engineered product or a cosmetic.
